# EUROPÄISCHE PATENTSCHRIFT

(11) **EP 0 969 834 B1**
(45) Veröffentlichungstag und Bekanntmachung des Hinweises auf die Patenterteilung: **02.05.2003**
(21) Anmeldenummer: 98916811.7
(22) Anmeldetag: 24.02.1998
(51) Int. Cl.: A61K 31/425, A61P 11/12

(54) **VERWENDUNG VON 2-METHYL-THIAZOLIDIN-2,4-DICARBONSÄURE ALS MUKOLYTIKUM**
USE OF 2-METHYLTHIAZOLIDINE-2,4-DICARBOXYLIC ACID AS MUCOLYTIC AGENT
UTILISATION D'ACIDE 2-METHYLTHIAZOLIDINE-2,4-DICARBOXYLIQUE COMME AGENT MUCOLYTIQUE

(30) Priorität: 03.03.1997 DE 19711052
(43) Veröffentlichungstag der Anmeldung: 12.01.2000
(73) Patentinhaber: Susilo, Rudy, 50937 Köln (DE)
(72) Erfinder: SUSILO, Rudy, D-50937 Köln (DE); WLODEK, Lidia, PL-30-041 Karkow (PL)
(74) Vertreter: Wablat, Wolfgang, Dr.Dr.
(86) Internationale Anmeldenummer: DE9800585
(87) Internationale Veröffentlichungsnummer: WO98038995

(56) Entgegenhaltungen:
- EP-A- 0 254 354
- DE-A- 2 116 629
- L. WLODEK ET AL.: "The effect of 2-substitute thiazolidine-4(R)-carboxylic acids on non-protein sulphydryl levels and sulphurtransferase activities in mouse liver and brain." BIOCHEM. PHARMACOL., Bd. 46, Nr. 1, 1993, Seiten 190-193, XP002068563
- L. WLODEK ET AL: "The antioxidative properties of thiazolidine derivatives." POL. J. PHARMACOL. PHARM., Bd. 41, Nr. 4, 1989, Seiten 369-375, XP002068564

## Beschreibung

Die Erfindung betrifft die Verwendung von 2-Methylthiazolidin-2,4-dicarbonsäure und/oder ihrer physiologisch verträglichen Salze zur Herstellung eines Mukolytikums.

Als Mukolytika werden in Deutschland N-Acetylcystein und 2-Mercaptoethansulfonat verwendet, wobei N-Acetylcystein das am meisten angewandte Mukolytikum darstellt. Die medizinische Indikation betrifft Erkrankungen der Atemwege mit starker Sekretion eines zähen Schleims, wie z. B. akuter und chronischer Bronchitis, Bronchiektasie, asthmoide Bronchitis, Asthma bronchiale, Bronchiolitis und Mukoviszidose.

Bezüglich des mukolytischen Wirkungsmechanismus des nukleophilen N-Acetylcysteins werden zwei Mechanismen diskutiert. Zum einen kann L-Cystein freigesetzt werden und zum anderen kann die freie Sulfhydrylgruppe der unveränderten Substanz direkt die Disulfidbrücken der Mukoproteine spalten. In der Folge kommt es zu einer Fraktionierung der Schleimbestandteile, eine Voraussetzung für die Abnahme der Viskosität und den besseren Abtransport des Schleims.

Die Freisetzung von L-Cystein erfolgt nur zu einem geringen Teil durch Hydrolyse, vorwiegend durch eine Aminosäuren-N-Deacylase, die beispielsweise im Cytosol von Leberzellen nachgewiesen werden konnte (Wlodek, L., Rommelspacher, H., Susilo, R., Radomski, J. und Hefle, G., Biochem. Pharmacol. 46: 917-928 (1993)).

Allgemein wird davon ausgegangen, dass es sich bei N-Acetylcystein um ein Medikament geringer Toxizität handelt. Allerdings gibt es einige, wenig beachtete Berichte, dass das Toxizitätsrisiko von N-Acetylcystein unterschätzt wird (Estrela, J.M., Saez, G.T., Such, L. und Vina, J., Biochem. Pharmacol. 32: 3483-3485 (1983) und Vina, J., Romero, F.J., Saez, G.T. und Pallardo, F.V., Experientia 39: 164-165 (1983)). Wegen des Risikos toxischer Reaktionen von N-Acetylcystein wurde immer wieder nach Alternativen gesucht. Die Applikation von L-Cystein selbst verbietet sich, da die Aminosäure sehr toxisch ist und zum Absterben von Hirnzellen führt (Karlsen, R.L., Grofova, Y., Malthe-Sorensen, D. und Farnum, E., Exp. Brain. Res. 208: 167-180 (1981)). Eine Möglichkeit, diese Toxizität zu umgehen, ist die Applikation einer sogenannten "pro-drug", also eines Vorläufermedikaments, aus der die wirksame Aminosäure erst im Körper kontrolliert freigesetzt wird.

Die Kondensation von carbonylhaltigen Stoffen mit L-Cystein zu Thiazolidinen wurde bereits beschrieben (Susilo, R., Rommelspacher, F. und Hoefle, G., J. Neurochem. 52: 1793-1800 (1989)).
Wichtig ist dabei, dass die Thiazolidine ein Reservoir für L-Cystein bilden, aus denen bei Bedarf die Aminosäure freigesetzt wird. Ein strukturell einfaches Thiazolidin ist das Kondensationsprodukt zwischen Formaldehyd und L-Cystein. Metaboliten dieser Substanz stellten sich allerdings als neurotoxisch heraus. Das Kondensationsprodukt zwischen Acetaldehyd und L-Cystein ist als ein Vorläufermedikament ebenfalls ungeeignet, da es unter physiologischen Bedingungen leicht in seine Komponenten zerfällt (Wlodek, L., Rommelspacher, H., Susilo, R., Radomski, J. und Hefle, G., Biochem. Pharmacol. 46: 917-928 (1993)).
2-Methyl-thiazolidin-2,4-dicarbonsäure wird im Stand der Technik verschiedentlich beschrieben. So findet sich in der DE 21 16 629 A die Verwendung von 2-Methylthiazolidin-2,4-dicarbonsäure als Leberschutzpräparat bzw. Arzneimittel. Ein entsprechender hepaprotektiver Effekt wird zudem in einer Studie an Ratten (Biochem. Pharmacol., 1993, Bd. 46, Nr. 1, S. 190-193) offenbart. In einer weiteren Studie (Pol. J. Pharmacol. Pharm., 1989, Bd. 41, Nr. 4, S. 369-375) wird ein antioxidativer Effekt der 2-Methyl-thiazolidin-2,4-dicarbonsäure untersucht. Einen Hinweis auf eine mukolytische Wirkung von Thiazolidin-4-carboxylsäure und verschiedenen davon abgeleiteten Derivaten, die jedoch aufgrund Ihrer Struktur keinen Hinweis auf eine entsprechende Wirkung der 2-Methyl-thiazolidin-2,4-dicarbonsäure geben, findet sich in der EP 0 254 354 A1.

Aufgabe der vorliegenden Erfindung ist es, eine physiologisch gut verträgliche Substanz zur Herstellung eines Mukolytikums zur Verfügung zu stellen, die zur Therapie von mit starker Schleimsekretion einhergehenden Erkrankungen der Luft- und Atemwege geeignet ist und welche weniger Nebenwirkungen aufweist als die bisher verwendeten Substanzen des Standes der Technik.

Diese Aufgabe wird erfindungsgemäß durch die Verwendung von 2-Methyl-thiazolidin-2,4-dicarbonsäure und/oder ihrer physiologisch verträglichen Salze zur Herstellung eines Mukolytikums gelöst.

Überraschenderweise wurde gefunden, dass 2-Methylthiazolidin-2,4-dicarbonsäure oder ihre physiologisch verträglichen Salze Mukolytika sind.

Die Synthese von 2-Methyl-thiazolidin-2,4-dicarbonsäure, seine Verwendung als Hepaprotektivum, sowie die Herstellung 2-Methyl-thiazolidin-2,4-dicarbonsäure enthaltender Arzneimittel in Form von Dragees oder Salben sind aus DE-OS 21 16 629 bekannt. Über die mukolytischen Eigenschaften von 2-Methyl-thiazolidin-2,4-dicarbonsäure und seiner physiologisch verträglichen Salze war bisher nichts bekannt.

Bevorzugt ist die Verwendung von 2-Methyl-thiazolidin-2,4-dicarbonsäure und/oder ihrer physiologisch verträglichen Salze zur Herstellung eines Arzneimittels zur Therapie von mit starker Schleimsekretion einhergehenden Erkrankungen der Luft- und Atemwege.

Insbesondere bevorzugt ist die Verwendung zur Herstellung eines Arzneimittels zur Therapie von Erkrankungen der Atemwege mit starker Sekretion eines zähen Schleims, wie z. B. akuter und chronischer Bronchitis, Bronchiektasie, asthmoide Bronchitis, Asthma bronchiale, Bronchiolitis und Mukoviszidose.

2-Methyl-thiazolidin-2,4-dicarbonsäure setzt überraschenderweise die Viskosität von Bronchialsekret herab. Bei vergleichsweise dünnflüssigem Schleim weisen äquimolare Konzentrationen von 2-Methyl-thiazolidin-2,4-dicarbonsäure und N-Acetylcystein eine ähnliche Wirksamkeit auf. Da die bisherigen Untersuchungen in Phosphatpuffer durchgeführt wurden, die SH-Gruppe der Thiazolidinderivate jedoch besonders durch cytosolische und membrangebundene Enzyme aktiviert wird (Susilo, R., Rommelspacher, F. und Hoefle, G., J. Neurochem. 52: 1793-1800 (1989) und Wlodek, L., Rommelspacher, H., Susilo, R., Radomski, J. und Hefle, G., Biochem. Pharmacol. 46: 917-928 (1993)), kann angenommen werden, dass die mukolytische Aktivität von 2-Methyl-thiazolidin-2,4-dicarbonsäure *in vivo* sogar noch wesentlich stärker ist.

Überraschenderweise wurde ferner gefunden, dass 2-Methylthiazolidin-2,4-dicarbonsäure eine Reduktion der Bildung der freien Radikale und eine Erhöhung der Sulfhydrylgruppen-Konzentration im Organismus hervorruft. Dies führt zu einer cytoprotektiven und entzündungshemmenden Wirkung dieser Verbindung. Dadurch ist diese Substanz den bisher bekannten Verbindungen, wie N-Acetylcystein deutlich überlegen. Die vom N-Acetylcystein bekannten toxischen Nebenwirkungen können durch die Verwendung von von 2-Methyl-thiazolidin-2,4-dicarbonsäure als Mukolytikum deutlich verringert werden.

Bei der Freisetzung von L-Cystein aus 2-Methylthiazolidin-2,4-dicarbonsäure entsteht als Nebenprodukt Pyruvat, eine physiologische Substanz, die völlig unschädlich ist. 2-Methyl-thiazolidin-2,4-dicarbonsäure zeichnet sich deshalb im Gegensatz zu N-Acetylcystein durch eine sehr gute Verträglichkeit aus. Es gibt sogar Hinweise auf eine protektive Wirkung von Pyruvat (Rastellini, C., Cicalese, L., Zeevi, A., Mattes, C., Stauko, R.T., Starzl, T.E. und Rao, A.S., Transplant. Proceed. 27: 3383-3384 (1995)). Pyruvat entsteht physiologischerweise aus Glukose und wird im Zitronensäurezyklus zur Energiegewinnung der Zelle benötigt. Aufgrund dieser Tatsache ist zu erwarten, dass durch eine langsame enzymatische Freisetzung von L-Cystein in den Körperzellen bzw. auch den Bronchien eine retardierende Wirkung erfolgt, die eine im Vergleich mit N-Acetylcystein länger anhaltende Wirkung erwarten lässt.

Die erfindungsgemäß verwendeten Salze von 2-Methylthiazolidin-2,4-dicarbonsäure werden in an sich bekannter Weise durch Umsetzung von 2-Methyl-thiazolidin-2,4-dicarbonsäure mit der entsprechenden Base erhalten.

Die gemäß der erfindungsgemäßen Verwendung hergestellten Arzneimittel bzw. Mukolytika enthalten neben 2-Methylthiazolidin-2,4-dicarbonsäure und/oder ihre physiologisch verträglichen Salze übliche Träger- und Verdünnungsmittel. Die Arzneimittel können zur oralen, rektalen, subcutanen, intravenösen oder intramuskulären Applikation oder zur Inhalation vorgesehen sein.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation bzw. zur Inhalation geeignet ist. Solche Darreichungsformen sind beispielsweise Tabletten, Filmtabletten, Dragees, Kapseln, Pillen, Pulver, Lösungen oder Suspensionen oder Depotformen oder Inhalationslösungen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Das folgende Beispiel erläutert die Erfindung:

### Beispiel 1:

### Mukolytische Aktivität von 2-Methyl-thiazolidin-2,4-dicarbonsäure

2-Methyl-thiazolidin-2,4-dicarbonsäure wurde nach der bekannten Methode von Schubert (Schubert, M. P., J. Biol. Chem. 114: 341-350 (1936)) synthetisiert.

Vierzehn Proben von Patienten wurden untersucht. Die medizinischen Diagnosen lauteten:
- zystische Fibrose (n = 5)
- Bronchiektasen (n = 2)
- chronische, spastische Bronchitis (n = 7)

Sieben von vierzehn Patienten erhielten N-Acetylcystein. Bei diesen Patienten wurde eine Karenz von mindestens fünf Stunden vor Entnahme des Schleims eingehalten. Da die Viskosität der Proben sehr unterschiedlich war, wurde die Viskosität eines Aliquots in einer Voruntersuchung bestimmt und die Proben soweit verdünnt, dass die Viskosität bei 40 pro Sekunde Schergeschwindigkeit (Geschwindigkeitsgefälle) unter 100 mPa * s lag. Die Endkonzentration der Proben lag zwischen 60 und 80% des Ausgangswertes.

Als Viskosimeter wurde das RheoStress 100 Meßsystem (Haake, Karlsruhe) verwendet. Als Meßkegel wurde der Typ C60/1 eingesetzt, mit einem Winkel von einem Grad und einem Radius von 30 mm.

Die Untersuchungen wurden teilweise mit Proben einer einzelnen Person durchgeführt, teilweise wurden die Proben von bis zu sechs Probanden vereinigt. Aliquots der einzelnen Personen wurden entweder mit Lösungsmittel (Natriumphosphatpuffer, 50 mM, pH 7,0; Kontrollproben) oder mit 15 mM oder 30 mM 2-Methyl-thiazolidin-2,4-dicarbonsäure oder mit 15 mM oder 30 mM N-Acetylcystein (Referenzsubstanz) versetzt. Die gekühlten Proben wurden vorsichtig geschüttelt und dann 15 min bei 37°C in einem Wasserbad inkubiert. Anschließend erfolgte die Messung mit dem Viskosimeter.

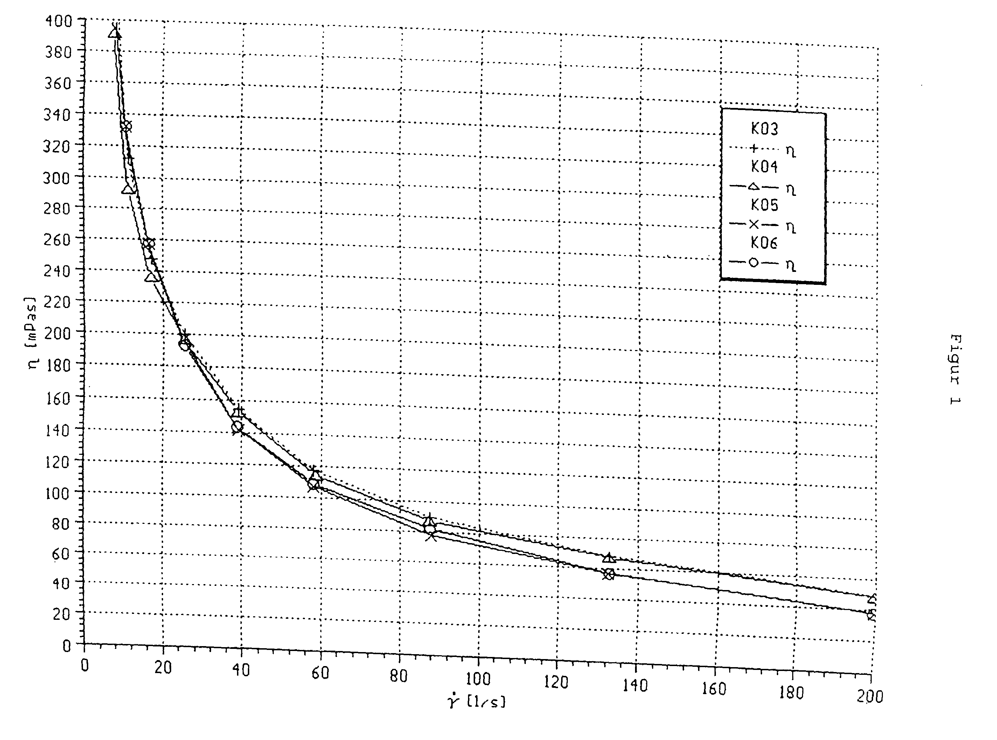

Die Ergebnisse der Messungen sind in Tabelle 1 und den Figuren 1 bis 5 dargestellt.

**Tabelle 1:**

| Effekt von Mukolytika auf die Viskosität von Bronchialsekret | | | | | |
|---|---|---|---|---|---|
| Probe | Konzentration Mukolytikum (mM) | Viskosität (mPa * s) | Probe | Konzentration Mukolytikum (mM) | Viskosität (mPa * s) |
| KO-1 | 0 | 150,3 | KO-2 | 0 | 11,0 |
| CP-1 | 15 | 134,0 | CP-2 | 15 | 6,7 |
| CP-1 | 30 | 108,2 | CP-2 | 30 | 8,1 |
| NAC-1 | 15 | 71,2 | NAC-2 | 15 | 7,1 |
| NAC-1 | 30 | 86,0 | NAC-2 | 30 | 8,8 |

Für die Probe Nr.1 wurden die Proben von sechs Patienten vereinigt. Die Werte stellen Mittelwerte von sechs (Kontrolle) bzw. jeweils vier Messungen dar. Die Werte mit Testsubstanzen sind signifikant weniger viskös als die Kontrollproben (p < 0,01). Probe Nr. 2 ist die Probe eines einzelnen Patienten.
- KO :: Kontrollprobe
- CP :: 2-Methyl-thiazolidin-2,4-dicarbonsäure
- NAC:: N-Acetylcystein

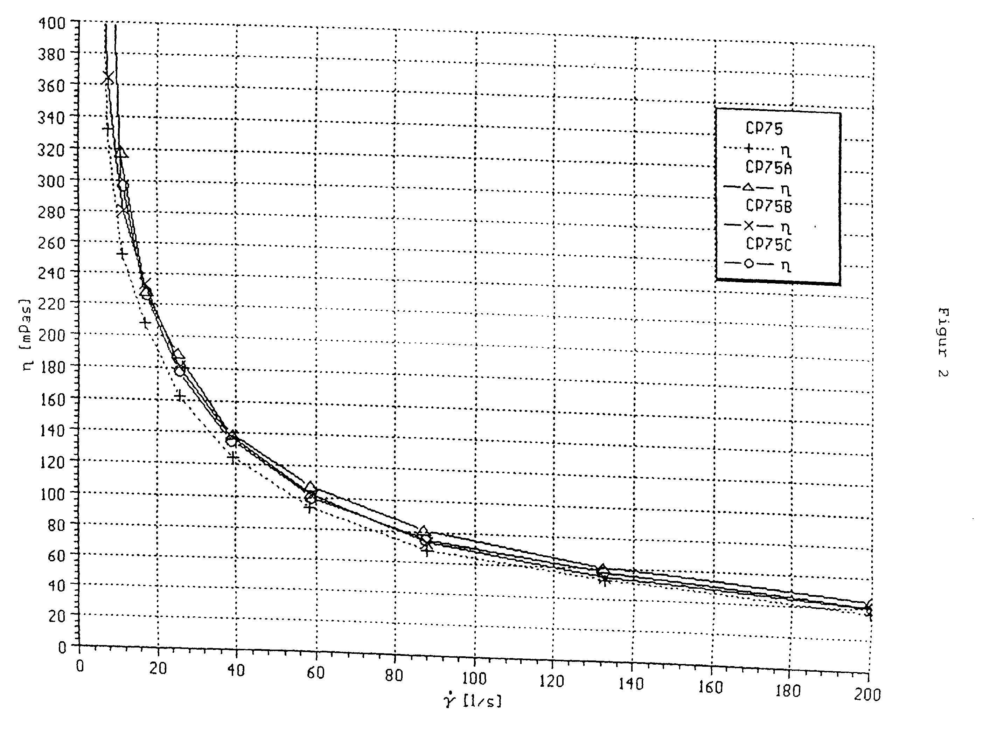

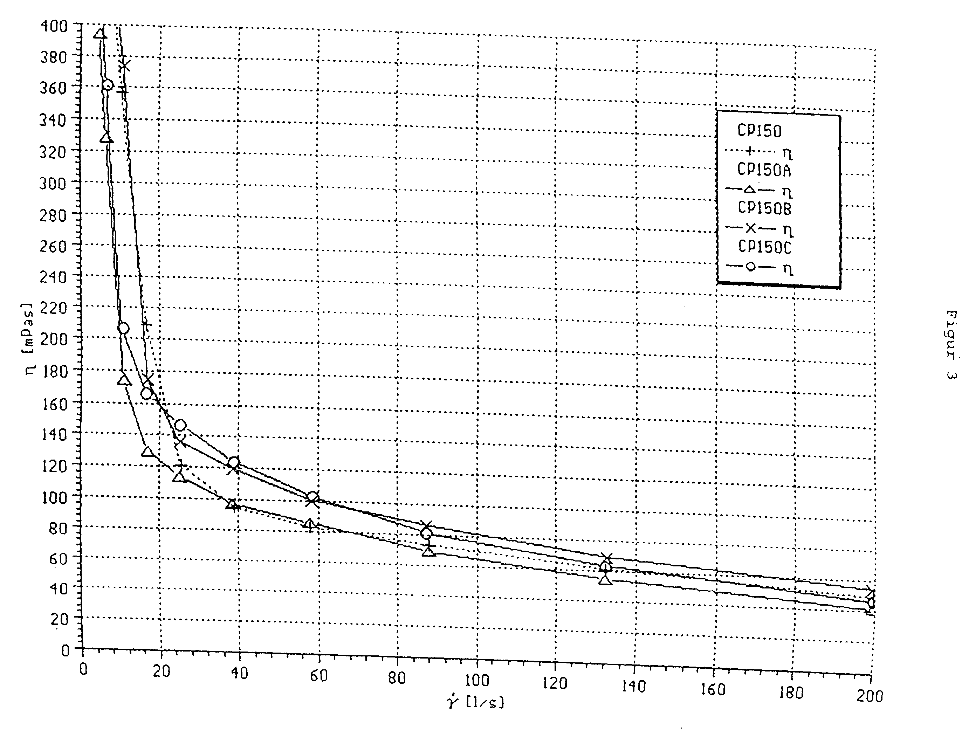

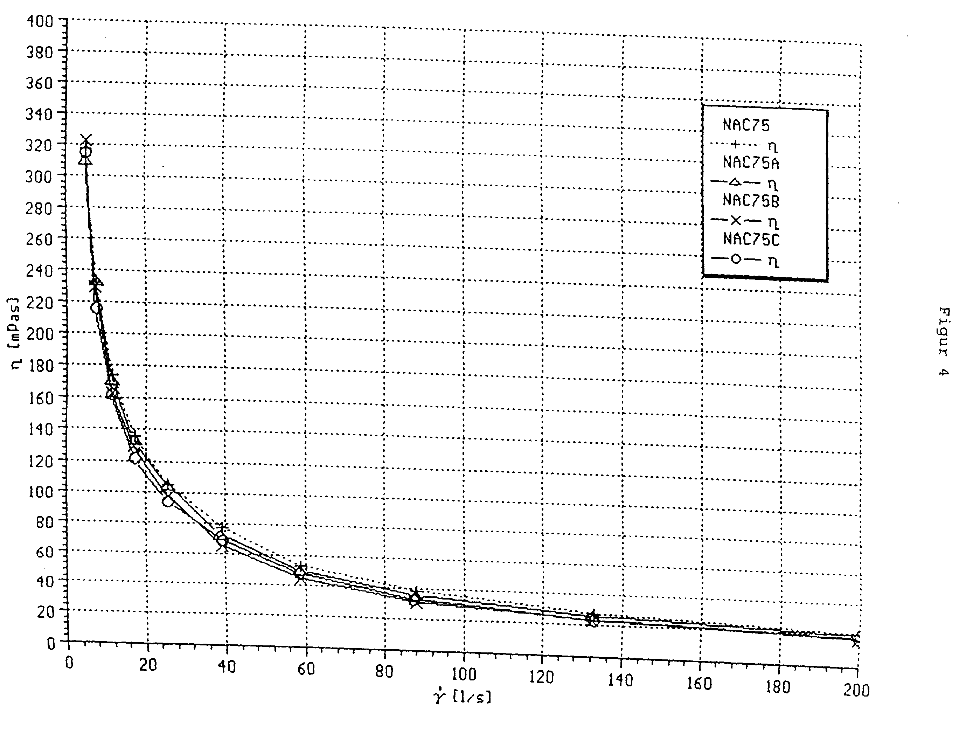

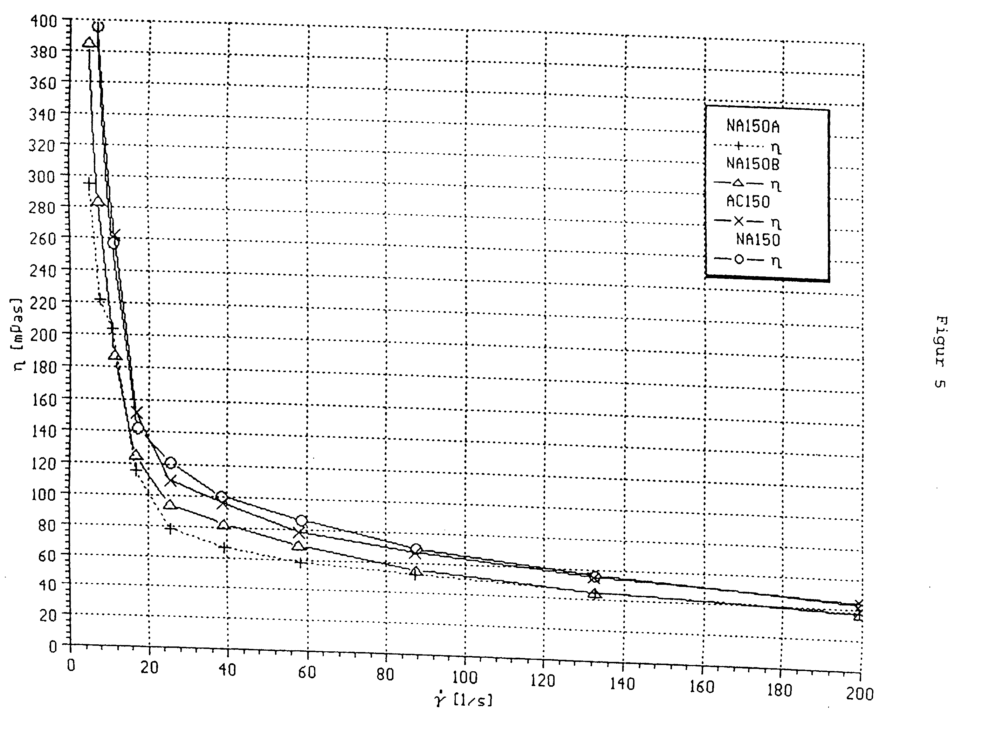

In Figur 1 bis 5 sind Diagramme dargestellt, wobei auf der Abszisse die Schergeschwindigkeit und auf der Ordinate die Viskosität aufgetragen ist. Der Wert für die Kontrollproben (Fig. 1) beträgt im Mittel 150 mPa * s (n = 6; nur vier Beispiele sind dargestellt). 15 mM CP führte zur signifikanten Abnahme der Viskosität (Fig. 2; 134 mPa * s). Dieser Effekt wird noch deutlicher bei Zugabe von 30 mM CP (Fig. 3; 108 mPa * s). Die Effekte der äquimolaren Konzentrationen von N-Acetylcystein sind ausgeprägter und betragen 71, bzw. 86 mPa * s (Fig. 4 und 5). Daraus kann geschlossen werden, dass bereits bei einer Konzentration von 15 mM N-Acetylcystein ein maximaler Effekt erzielt wird. Offenbar hängt die Effizienz der Mukolytika stark von der Ausgangsviskosität der Probe ab. In einer weiteren Probe mit einer wesentlich geringeren Viskosität (11 mPa * s) konnte gefunden werden, dass 15 mM N-Acetylcystein zu einer Verminderung der Viskosität auf 7,1 mPa * s führte und 30 mM N-Acetylcystein auf 8,8 mPa * s (Tab. 1). Dies bestätigt den Befund mit der anderen Probe, dass 15 mM N-Acetylcystein bereits einen maximalen Effekt erzielt. Interessanterweise konnte beobachtet werden, dass 15 mM des Thiazolidinderivates zu einer Verminderung der Viskosität in der gleichen Größenordnung führte (6,7 mPa * s) und dieser Effekt durch eine Verdoppelung der Konzentration nicht verstärkt werden konnte (30 mM; 8,1 mPa * s).

### Kurze Beschreibung der Zeichnungen:

### Figur 1:

Viskosität von vier Kontrollproben (K03 bis K06). Auf der x-Achse ist die Schergeschwindigkeit (γ) und auf der Y-Achse die Viskosität (η) aufgetragen.

### Figur 2:

Viskosität von vier Proben (CP75, CP75A, CP75B und CP75C) aus demselben Pool wie in Abb. 1, die eine Konzentration von 15 mM 2-Methyl-thiazolidin-2,4-dicarbonsäure (CP) enthalten. Auf der x-Achse ist die Schergeschwindigkeit (γ) und auf der Y-Achse die Viskosität (η) aufgetragen.

### Figur 3:

Viskosität von vier Proben (CP150, CP150A, CP150B und CP150C) aus demselben Pool wie in Abb. 1, die eine Konzentration von 30 mM 2-Methyl-thiazolidin-2,4-dicarbonsäure (CP) enthalten. Auf der x-Achse ist die Schergeschwindigkeit (γ) und auf der Y-Achse die Viskosität (η) aufgetragen.

### Figur 4:

Viskosität von vier Proben (NAC75, NAC75A, NAC75B und NAC75C) aus demselben Pool wie in Abb. 1, die eine Konzentration von 15 mM N-Acetylcystein (NAC) enthalten. Auf der x-Achse ist die Schergeschwindigkeit (γ) und auf der Y-Achse die Viskosität (η) aufgetragen.

### Figur 5:

Viskosität von vier Proben (NAC150, NAC150A, NAC150B und NAC150C) aus demselben Pool wie in Abb. 1, die eine Konzentration von 30 mM N-Acetylcystein (NAC) enthalten. Auf der x-Achse ist die Schergeschwindigkeit (γ) und auf der Y-Achse die Viskosität (η) aufgetragen.

## Patentansprüche

1. Verwendung von 2-Methyl-thiazolidin-2,4-dicarbonsäure und/oder ihrer physiologisch verträglichen Salze zur Herstellung eines Mukolytikums.

2. Verwendung von 2-Methyl-thiazolidin-2,4-dicarbonsäure und/oder ihrer physiologisch verträglichen Salze nach Anspruch 1 zur Herstellung eines Arzneimittels zur Behandlung von mit starker Schleimsekretion einhergehenden Erkrankungen der Luft- und Atemwege.

3. Verwendung von 2-Methyl-thiazolidin-2,4-dicarbonsäure und/oder ihrer physiologisch verträglichen Salze nach Anspruch 1 oder 2 zur Herstellung eines Arzneimittels zur Behandlung von akuter und chronischer Bronchitis, Bronchiektasie, asthmoider Bronchitis, Asthma bronchiale, Bronchiolitis oder Mukoviszidose.

## Claims

1. Use of 2-methyl-thiazolidine-2,4-dicarboxylic acid and/or its physiologically tolerable salts for the manufacture of a mucolytic agent.

2. Use of 2-methyl-thiazolidine-2,4-dicarboxylic acid and/or its physiologically tolerable salts according to claim 1 for the manufacture of an agent for treatment of diseases of the respiratory tract that are accompanied by intense secretion of mucus.

3. Use of 2-methyl-thiazolidine-2,4-dicarboxylic acid and/or its physiologically tolerable salts according to claims 1 or 2 for the manufacture of an agent for treatment of acute and chronic bronchitis, bronchiectasis, asthmoid bronchitis, bronchial asthma, bronchiolitis, and mucoviscidosis.

## Revendications

1. Utilisation de l'acide 2-méthyl-thiazolidine-2,4-dicarboxylique et/ou de ses sels physiologiquement acceptables pour la préparation d'un mucolytique.

2. Utilisation de l'acide 2-méthyl-thiazolidine-2,4-dicarboxylique et/ou de ses sels physiologiquement acceptables selon la revendication 1 pour la préparation d'un médicament pour le traitement des maladies des voies aériennes et respiratoires s'accompagnant de fortes sécrétions de mucus.

3. Utilisation de l'acide 2-méthyl-thiazolidine-2,4-dicarboxylique et/ou de ses sels physiologiquement acceptables selon la revendication 1 ou 2 pour la préparation d'un médicament pour le traitement de la bronchite aiguë et chronique, de la bronchiectasie, de la bronchite asthmoïdale, de l'asthme bronchique, de la bronchiolite ou de la mucoviscidose.
